# EUROPÄISCHE PATENTANMELDUNG

(11) **EP 4 360 612 A1**
(43) Veröffentlichungstag der Anmeldung: **01.05.2024**
(21) Anmeldenummer: 23197072.4
(22) Anmeldetag: 13.09.2023
(51) Int. Cl.: A61K 8/04, A61K 8/44, A61K 8/60, A61K 8/73, A61Q 19/10

(54) **KOSMETISCHES REINIGUNGSGEL MIT HOHER TRANSPARENZ**

(30) Priorität: 25.10.2022 DE 102022211303
(71) Anmelder: Beiersdorf AG, 22529 Hamburg (DE)
(72) Erfinder: Heins, Sabine, 25462 Rellingen (DE); Rübenhaus, Philipp, 22399 Hamburg (DE); Triller, Benjamin, 22941 Jersbek (DE)
(74) Vertreter: Beiersdorf AG

(57) **Zusammenfassung**

Reinigungsgel mit einer hohen Transparenz, das derart ausgestaltet ist, dass eine Entnahme aus einer Tube erfolgen kann. Das Reinigungsgel ist auch zur Rasurvorbereitung vorgesehen.

## Beschreibung

Die vorliegende Erfindung beschreibt ein kosmetisches Reinigungsgel mit einer hohen Transparenz, das derart ausgestaltet ist, dass eine Entnahme aus einer Tube erfolgen kann. Das Reinigungsgel ist auch zur Rasurvorbereitung vorgesehen.

Reinigungsgele sind an sich bekannt und auch transparente Reinigungsgele sind vielfach erhältlich. Um die Transparenz zu verdeutlichen, werden derartige Zubereitungen zumeist in transparenten Verpackungen angeboten. Diese Verpackungen sind in der Regel Plastikflaschen, bei denen die Entnahme aus einer dafür vorgesehenen Öffnung erfolgt. Als Beispiele seien die folgenden Produkte genannt: Jasmine Scent Nourishing Shower (Mintel Eintragsnummer: 8948416); Cotton Blossom Scented Care Shower (Mintel Eintragsnummer: 8413185) und Shower Gel with Lemongrass & Oil (Mintel Eintragsnummer: 567965).

Reinigungsprodukte, auch Reinigungsgele, dienen dazu, die Haut und die Haare zu reinigen. Nach dem Reinigungsprozess stellt sich beim Anwender im Idealfall ein Gefühl von Sauberkeit, Frische und Wohlbefinden ein. Der Mensch hat von je her das Bedürfnis gehabt sich zu reinigen, d.h. Schmutz von der Haut oder aus den Haaren zu entfernen. Ziel des Reinigungsprozesses ist es Schmutz von der Hautoberfläche zu entfernen. Der Schmutzfilm besteht aus festen oder flüssigen Komponenten, die von außen auf die Haut gelangt sind. Ebenfalls zum Schmutzfilm gehören überschüssige Hautlipide und abgestorbene Körperzellen. Mithilfe von oberflächenaktiven Inhaltsstoffen in den Reinigungszusammensetzungen gelangen die Komponenten des Schmutzfilms in die Waschflotte und werden durch den Abspülprozess von der Haut entfernt. Neben der Beseitigung des Schmutzfilms können jedoch auch in begrenztem Maße Oberflächenlipide der Haut, beispielsweise Barrierelipide, durch die oberflächenaktiven Substanzen in den Reinigungszubereitungen gelöst und im Abspülvorgang mit entfernt werden. Der Verlust der Hautlipide kann zu Austrocknungserscheinungen und Hautirritationen führen.

Es sind verschiedene Konzepte bekannt, um in Reinigungszubereitungen die irritative Wirkungen von bestimmten Tensiden abzumildern. Eine Möglichkeit besteht in der Auswahl von besonders milden Tensiden. Eine weitere Möglichkeit bietet der Einsatz von rückfettenden Inhaltsstoffen in Reinigungszubereitungen. Ebenso trägt der Verzicht von anionischen Tensiden auf Seifen-Basis dazu bei, das Reizpotential von Reinigungszubereitungen abzusenken.

Über die Zeit haben sich die Reinigungsprozesse gewandelt wie auch die Produkte, die zur Reinigung verwendet werden. Heutzutage werden vielfach spezielle Reinigungsprodukte verwendet, z.B. für den Körper, für die Haare, für das Gesicht, für die Füße und andere mehr. Die jeweiligen Reinigungsprodukte werden wiederum in unterschiedlichen Ausführungsformen angeboten.

Trotz dieser Vielzahl von verschiedenen Produktformen, wird bei Verbraucherumfragen von den Konsumenten immer wieder der Wunsch nach neuen, innovativen Produktformen geäu-βert.

Somit bestand also der Bedarf, eine Reinigungszubereitung in einer andersartigen Verpackung zur Verfügung zu stellen.

Das Reinigungsgel sollte transparent sein, idealerweise sehr transparent. Transparenz wird von Verbraucher mit Frische und Sauberkeit assoziiert, beides Eigenschaften, die ein Reinigungsprodukt nach der Anwendung beim Verbraucher erreichen soll und muss. Um die Transparenz sichtbar zu machen und eine andersartige Verpackungseinheit zur Verfügung zu stellen, bestand die Aufgabe darin, das erfindungsgemäße Reinigungsgel in einer transparenten Tube anzubieten.

Reinigungszubereitungen, auch Reinigungsgele, die in einer Tube angeboten werden, sind bereits erhältlich. Die Tuben sind jedoch in den allermeisten Fällen undurchsichtig, also nicht transparent. Als Beispiele seien die folgenden Produkte genannt: 2in1 Deep Cleanse Gel (Mintel-Eintragsnummer: 9754606), Cleansing Gel (Mintel-Eintragsnummer 9769706) und andere mehr.

Für die Entnahme eines Reinigungsgels aus einer Tube gelten besondere Anforderungen. Die Zubereitung muss bestimmte rheologische Eigenschaften aufweisen, um eine Problemlose Entnahme aus der Tube sicherzustellen.

Weiterhin sollte auf bestimmte Substanzen verzichtet werden, wie der Einsatz von synthetischen Polymeren, von Parabenen und der Einsatz von Sulfat-haltigen Verbindungen.

Der Verzicht auf synthetische Polymere bedeutet, dass wohl bekannte und gut einsetzbare Polymere aus der Gruppe der Acrylat-basierten Polymere nicht mehr verwendet werden; gleichwohl sollen aber Produkte mit vergleichbaren rheologische und sensorischen Eigenschaften bereitgestellt werden.

Auch der Verzicht auf Parabene, die sich als Konservierungsmittel in Kosmetika bewährt haben, gleichwohl aber von Verbraucherinnen nicht mehr gewünscht werden, führt dazu alternative Mittel zur Konservierung von Kosmetika einzusetzen, die aber die Anforderungen der EU an die Haltbarkeit von Kosmetika erfüllen müssen.

In Bezug auf den Verzicht von Sulfat-haltigen Verbindungen muss auch eine Alternative für das vielfach in Reinigungszubereitungen eingesetzte Tensid Natrium Laurylethersulfat gefunden werden. Dieses Tensid zeichnet sich durch eine gute Reinigungsleistung und gute Schaumbildung aus.

Weiterhin sollen die Reinigungsgele mild zur Haut sein und einen pH-Wert im neutralen oder Haut-neutralen Bereich aufweisen.

Gelöst wurden alle diese Aufgaben durch ein kosmetisches Reinigungsgel, enthaltend
- wenigstens ein Aminosäure-Tensid, bevorzugt ausgewählt aus wenigstens einem Natrium Acyl-Sarkosinat und/oder wenigstens einem Natrium/Dinatrium Acyl Glutamat,
- wenigstens ein weiteres Tensid, das kein Aminosäure-Tensid ist, bevorzugt ausgewählt aus wenigstens einem Alkylpropylbetain und/oder wenigstens einem Alkylpolyglucosid,
- eine Kombination der Polymere Xanthan Gum und Hydroxypropyl Guar.

Das erfindungsgemäße Reinigungsgel ist eine wässrige Zubereitung. Der Wassergehalt beträgt von 50 bis 95 Gew.-%, bevorzugt von 65 bis 90 Gew.-%, bezogen auf das Gesamtgewicht des Reinigungsgels.

Das erfindungsgemäße Reinigungsgel ist auch zur Rasurvorbereitung vorgesehen.

Unter Rasurvorbereitung wird ein Vorgehen, insbesondere bei der Nassrasur, verstanden, dass dazu führt, dass die (Bart-)Haare aufgeweicht werden, so dass der Kraftaufwand bei der Rasur verringert wird. Dadurch wird das Ziehen am Haarschaft ("Ziepen") vermindert. Das Aufweichen der (Bart-)Haare wird durch eine Wasseraufnahme erreicht. Die Sebumschicht der Haare muss aber entfernt werden, damit die Wasseraufnahme hinreichend gut und schnell erfolgen kann.

Erfindungsgmäß ist das Reinigungsgel frei von synthetischen Polymeren. Unter synthetischen Polymeren sind solche Polymere zu verstehen, die sich aus einem oder verschiedenen Monomeren zusammensetzen und die in einem chemischen, nicht von natürlichen Organismen ausgeführten Syntheseprozess entstehen. Diese Monomere sind in der Regel rein synthetischer Natur. Daraus folgt, dass das gesamte Polymer synthetischer Natur ist. Derartige Polymere haben kein natürliches Äquivalent und können daher auch nicht in einem biologischen Prozess abgebaut werden oder nur äußerst langsam. Dem erfindungsgemäßen Reinigungsgel werden keine synthetischen Polymere zugesetzt und sie gelangen auch nicht als Bei- oder Hilfsstoffe von anderen Rohstoffen in das Reinigungsgel. Das erfindungsgemäße Reinigungsgel ist also frei von synthetischen Polymeren.

Erfindungsgmäß ist das Reinigungsgel frei von Parabenen.

Unter Parabenen werden insbesondere die für kosmetische Zubereitungen zugelassenen Parabene, nämlich 4-Hydroxybenzoesäure, Butylparaben, Ethylparaben, Isobutylparaben, Isopropylparaben, Kalium Butylparaben, Kalium Ethylparaben, Kalium Methylparaben, Kalium Paraben, Kalium Propylparaben, Kalzium Paraben, Methylparaben, Natrium Butylparaben, Natrium Ethylparaben, Natrium Isobutylparaben, Natrium Methylparaben, Natrium Paraben, Natrium Propylparaben, Natrium Propylparaben, Phenylparaben und/oder Propylparaben verstanden.

Erfindungsgmäß ist das Reinigungsgel frei von Sulfat-haltigen Verbindungen.

Unter dem Begriff Sulfate werden Salze und Ester der Schwefelsäure verstanden. Die Salze sind durch die Formeln SO₄²⁻ (Anion) und HSO₄⁻ (Hydrogensulfat-Anion) darstellbar, während die Ester die allgemeine Formel R-O-SOz-O-R` haben, wobei R und/oder R` organische Reste sind und R` auch Wasserstoff sein. Bei entsprechendem pH-Wert dissoziiert die Säure und liegt als Salz vor.

Im Sinne der vorliegenden Erfindung bedeutet Sulfat-frei, dass der jeweilige Rohstoff oder die jeweilige Zusammensetzung weniger als 0,1 Gew. %, bevorzugt weniger als 0,01 Gew.-%, insbesondere bevorzugt 0 Gew. % an Sulfat-Ionen und/oder Estern der Schwefelsäure enthält.

Das erfindungsgemäße Reinigungsgel enthält wenigstens ein Aminosäure-Tensid. Diese Tenside zeichnen sich dadurch aus, dass Aminosäuren die Grundlage bilden, die mit Fettsäuren oder (natürlichen) Fettsäuregemischen reagiert haben. Vorteilhaft werden N-Acyl-Aminosäuren als Tenside in kosmetischen Zubereitungen eingesetzt.

Im erfindungsgemäßen Reinigungsgel sind weiter vorteilhaft wenigstens ein Natrium Acyl-Sarkosinate und/oder wenigstens ein Natrium/Dinatrium Acyl Glutamat enthalten.

Natrium Acylsarkosinate lassen sich durch die Formel

RCON(CH₃)CH₂COOM

beschreiben, wobei R ein Alkyl- oder Alkenylrest mit 5 bis 29 Kohlenstoffatomen, bevorzugt 7 bis 21 Kohlenstoffatomen und M ein Natriumkation ist. Weiter bevorzugt ist ein Acylsarkosinat mit einem Alkylrest von 11 Kohlenstoffatomen, also ein Lauroylsarkosinat. Das Lauroylsarkosinat liegt als Natrium Lauroylsarkosinat vor, das beispielsweise unter der Handelsbezeichnung Eversoft S-12 bei der Firma Sino Lion (USA) erhältlich ist.

Wenn in dem erfindungsgemäßen Reinigungsgel wenigstens ein Natrium Acylsarkosinat enthalten ist, so liegt es mit einem Gehalt von 0,5 bis 10,0 Gew.-%, bevorzugt 2,0 bis 6,0 Gew.-% vor, bezogen auf das Gesamtgewicht des Gels. Diese Angabe bezieht sich auf den Aktivgehalt .

Was unter "aktiver Einsatzkonzentration" bzw. "Aktivaehalt" im Sinne der vorliegenden Erfindung zu verstehen ist, soll das folgende Beispiel verdeutlichen: Liegt ein Rohstoff z. B. in Form einer 50 %-igen Lösung vor (beispielsweise, weil er nur in dieser Form auf dem Markt erhältlich ist), so ist - bei einer Einsatzkonzentration dieser "Rohstofflösung" von 8,0 Gew.-% - in der Zubereitung nur ein "Aktivgehalt" von 4,0 Gew.-%, bezogen auf das Gesamtgewicht der Zubereitung, verwirklicht.

Natrium/Dinatrium Acylglutamate lassen sich durch folgende Formel beschreiben,

RCONHCH(CH₂CH₂COOM_{A})COOM_{B}

wobei R ein Alkyl- oder Alkenylrest mit 5 bis 29 Kohlenstoffatomen, bevorzugt 7 bis 21 Kohlenstoffatomen und M_{A} im Falle von Natrium Acylglutamat ein Natriumkation und M_{B} ein Wasserstoffatom sind oder im Falle von Dinatrium Acylglutamat M_{A} und M_{B} jeweils ein Natriumkation sind. Weiter bevorzugt ist ein Acylglutamat mit einem Alkylrest, der sich von einem natürlichen Öl oder Fett ableitet, also beispielsweise Alkylreste von Fettsäuren wie sie in Palmöl, Sojaöl oder Kokosöl vorliegen. Ganz besonders bevorzugt ist die Verbindung Dinatrium Cocoyl Glutamat/Natrium Cocoyl Glutamat, die beispielsweise unter der Handelsbezeichnung Eversoft UCS-40S bei der Firma Sino Lion (USA) oder als Hostapon CCG von der Firma Clariant erhältlich ist.

Wenn in dem erfindungsgemäßen Reinigungsgel wenigstens ein Natrium/Dinatrium Acylglutamat enthalten ist, so liegt es mit einem Gesamtgehalt von 0,5 bis 10,0 Gew.-%, bevorzugt 0,5 bis 5,0 Gew.-% vor, bezogen auf das Gesamtgewicht des Gels. Diese Angabe bezieht sich auf den Aktivgehalt.

Wenn in dem erfindungsgemäßen Reinigungsgel wenigstens ein Natrium Acylsarkosinat und wenigstens ein Natrium/Dinatrium Acylglutamat vorliegen, so beträgt der Gesamtgehalt der genannten Tenside 1,0 bis 12,0 Gew.-%, bevorzugt 2,5 bis 11,0 Gew.-%, bezogen auf das Gesamtgewicht des Gels. Diese Angabe bezieht sich jeweils auf den Aktivgehalt.

Das erfindungsgemäße Reinigungsgel enthält wenigstens ein weiteres Tensid, das kein Aminosäure-Tensid ist, bevorzugt ausgewählt aus Alkylpropylbetainen und/oder Alkypolyglucosiden.

Alkylamidopropylbetaine lassen sich durch die folgende Struktur darstellen: 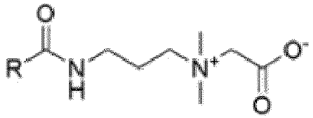 wobei R ein Alkylrest ist, der 7 bis 21 Kohlenstoffatome aufweist.

In einer bevorzugten Ausführungsform ist das Alkylamidopropylbetaine ein Cocamidopropylbetaine.

Cocamidopropylbetaine kann beispielsweise von der Firma Evonik unter der Handelsbezeichnung Tegobetain F50 B oder von der Firma Solvay unter der Handelsbezeichnung Mackam 50ULM bezogen werden.

Wenn in dem erfindungsgemäßen Reinigungsgel wenigstens ein Alkylamidopropylbetain enthalten ist, so liegt es mit einem Gesamtgewicht von 0,5 bis 15 Gew.-%, bevorzugt 2,5 bis 12 Gew.-% vor, bezogen auf das Gesamtgewicht des Gels. Diese Angabe bezieht sich auf den Aktivgehalt.

Alkylpolyglucoside lassen sich durch die folgende Formel beschreiben: 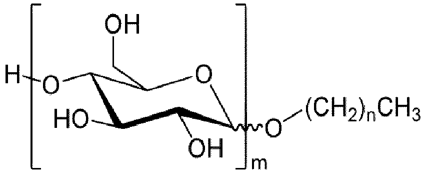 wobei m die Zahlen 1 bis 5 und n die Zahlen 5 bis 21, bevorzugt 9 bis 17 annehmen kann. Die durchschnittliche Anzahl an Glucose-Einheiten liegt bevorzugt bei 1,2 bis 2, weiter bevorzugt bei 1,2 bis 1,8.

Es ist bevorzugt, wenn das wenigstens eine Alkylpolyglucosid aus der Gruppe Coco-Glucosid, Decylglucosid, Laurylglucosid und/oder Caprylyl/Capryl Glucosid ausgewählt wird, insbesondere Cocoglucosid und/oder Caprylyl/Capryl Glucosid.

Cocoglucosid kann beispielsweise bei der Firma BASF unter der Handelsbezeichnung Plantacare 818 UP bezogen werden und Caprylyl/Capryl Glucosid kann beispielsweise bei der Firma Seppic GmbH unter der Handelsbezeichnung Oramix CG 110 bezogen werden.

Wenn in dem erfindungsgemäßen Reinigungsgel wenigstens ein Alkylpolyglucosid enthalten ist, so liegt es mit einem Gesamtgewicht von 0,1 bis 10,0 Gew.-%, bevorzugt 1,0 bis 2,5 Gew.-% vor, bezogen auf das Gesamtgewicht des Gels. Diese Angabe bezieht sich auf den Aktivgehalt.

Wenn in dem erfindungsgemäßen Reinigungsgel wenigstens ein Alkylamidopropylbetain und wenigstens ein Alkylpolyglucosid enthalten sind, so liegt das Gesamtgewicht der genannten Tenside im Bereich von 0,6 bis 15,0 Gew.-%, bevorzugt 2,6 bis 12,0 Gew.-%, bezogen auf das Gesamtgewicht des Gels. Diese Angabe bezieht sich auf den Aktivgehalt.

Das erfindungsgemäße Reinigungsgel enthält eine Kombination der Polymere Xanthan Gum und Hydroxypropyl Guar. Die genannte Kombination leistet einen wesentlichen Beitrag zur Transparenz und den wünschenswerten rheologischen Eigenschaften des erfindungsgemäßen Reinigungsgels.

Hydroxypropyl Guar ist der Sammelbegriff für die Reaktionsprodukte, die bei der alkalisch katalysierten Veretherung von Guarmehl mit Propylenoxid entstehen. Dabei werden die Hydroxywasserstoffatome des Guarans teilweise durch Gruppen des Typs 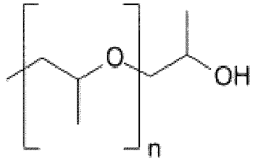 mit n≥ 0 ersetzt.

Hydroxypropyl Guar kann beispielsweise unter der Handelsbezeichnung Jaguar HP-105 bei der Firma Rhodia Operations SA bezogen werden.

Xanthan Gum wird mit Hilfe von Mikroorganismen der Gattung Xanthomonas gewonnen. Xanthan Gum ist ein Polymer, das aus einer Hauptkette aus β-1,4 glykosidisch verknüpften Glucosemolekülen besteht. An jedem zweiten Glucoserest hängt eine Seitenkette aus zwei Mannoseresten, einem Glucoronsäurerest und Pyruvat.

Xanthan Gum löst sich gut in heißem und kaltem Wasser, dabei bilden sich Einfachhelices und Doppelhelices. Es entsteht ein dreidimensionales Netzwerk.

Xanthan Gum kann beispielsweise unter der Handelsbezeichnung Keltrol CG-F V von der Firma CP Kelco bezogen werden.

In dem erfindungsgemäßen Reinigungsgel ist die Kombination der Polymere Xanthan Gum und Hydroxypropyl Guar mit einem Gesamtgewicht von 0,9 bis 3,0 Gew.-%, bevorzugt 1,0 bis 2,5 Gew.-% enthalten, bezogen auf das Gesamtgewicht des Gels. Dabei ist es vorteilhaft, wenn Xanthan Gum mit einem Gehalt von 0,8 bis 1,5 Gew.-% und Hydroxypropyl Guar mit einem Gehalt von 0,1 bis 1,5 Gew.-% enthalten ist, bezogen auf das Gesamtgewicht des Gels.

Da auf die Verwendung der Konservierungsmittelgruppe der Parabene verzichtet werden soll, muss die Konservierung des erfindungsgemäßen Reinigungsgels auf eine alternative Weise erfolgen. Was im Sinne der Erfindung unter Parabenen zu verstehen ist, wurde bereits beschrieben.

In dem erfindungsgemäßen Reinigungsgel kann das Konservierungsmittel Phenoxyethanol enthalten sein.

Phenoxyethanol kann durch folgende Formel dargestellt werden:

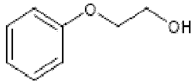

Phenoxyethanol ist vor allem in saurem und neutralem, aber auch im alkalischen Milieu wirksam und völlig ungiftig. Bereits durch den Einsatz von niedrigen Konzentrationen kann eine ausreichende Konservierung kosmetischer Produkte erreicht werden. Aufgrund seines neutralen Geschmacks fand Phenoxyethanol schnell Eingang in die pharmazeutische und kosmetische Industrie. Seine Wirkung richtet sich gegen Gram-positive und Gram-negative Bakterien, wie zum Beispiel Escherichia coli, Pseudomonas aeruginosa, Proteus mirabilis, Staphylococcus aureus, sowie gegen Hefepilze, wie Candida albicans und andere Mikroorganismen. Phenoxyethanol ist eine viskose Flüssigkeit von schwachem, leicht angenehmen Geruch und einem zusammenziehenden Geschmack. Es wurde in der Natur nachgewiesen in tropischen Früchten, in Cichorium endivia sowie in grünem Tee (Camellia sinesis). Es hat einen milden, rosenähnlichen Duft und wird für Parfümkompositionen auch als Fixatur eingesetzt. Es ist mischbar mit Aceton, Ethylalkohol und Glycerin, löslich in und Fetten, z.B. Oliven- und Erdnussöl, jedoch wenig löslich in Wasser.

Wenn im erfindungsgemäßen Reinigungsgel Phenoxyethanol enthalten ist, so liegt es mit einem Gehalt von 0,4 bis 0,95 Gew.-%, bevorzugt 0,6 bis 0,9 Gew.-% vor, bezogen auf das Gesamtgewicht des Gels.

Weiterhin kann das erfindungsgemäße Reinigungsgel wenigstens einen Stabilisator enthalten. Im Sinne der vorliegenden Erfindung sind als Stabilisatoren diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung für die entsprechende Zubereitung haben und/oder im gemeinsamen Einsatz mit Konservierungsmitteln die Stabilität der Zubereitung fördern.

Als Stabilisatoren sind folgende Verbindungen vorteilhaft: Propylenglycol, Ethylhexylglycerin, 1,2-Hexanediol, Methylpropanediol, Butylene Glycol, Caprylyl Glycol, Glyceryl Caprylate, Hydroxyacetophenone, Pentylene Glycol und/oder Ethanol, bevorzugt verwendet als Alkohol denat. Das Vergällungsmittel im Alkohol denat. ist vorteilhaft Isopropylalkohol und t-Butylalkohol. Weiter vorteilhaft werden die Stabilisatoren Methylpropandiol, Caprylyl Glycol und/oder Alkohol denat. verwendet.

Wenn in dem erfindungsgemäßen Reinigungsgel wenigstens ein Stabilisator enthalten ist, so liegt der wenigstens eine Stabilisator mit einem Gesamtgewicht von 0,2 bis 10,0 Gew.-% vor, bezogen auf das Gesamtgewicht des Gels.

Der pH-Wert des erfindungsgemäßen Reinigungsgels liegt im neutralen oder Haut-neutralen Bereich. Der optimale pH-Wert der Gesichts- und Körperhaut liegt zwischen 4,7 und 5,75; dies entspricht dem Haut-neutralen pH-Bereich. Ein pH-Wert von 7 gilt als neutral. Alles darunter ist sauer, alles darüber alkalisch. Das heißt, der natürliche pH-Wert der Haut liegt im sauren Bereich.

Der pH-Wert kann mit den im technischen Gebiet der Kosmetik üblichen Mitteln eingestellt und stabil gehalten werden. Vorteilhaft wird der pH-Wert jedoch mit einer physiologisch verträglichen organischen Säure, insbesondere vorteilhaft mit Citronensäure eingestellt.

Reinigungsgele, deren pH-Wert auf einen neutralen Wert (pH = 7) eingestellt werden, enthalten vorteilhaft als Stabilisatoren nur Alkohol denat. und Caprylyl Glycol und keine Konservierungsmittel.

Reinigungsgele, deren pH-Wert auf einen Haut-neutralen Wert (beispielsweise pH-Wert = 5,5) eingestellt wird, enthalten vorteilhaft als Aminosäuretensid nur Natrium Lauroyl Sarcosinate, als weitere Tenside nur Cocamidopropylbetaine und Cocoglucosid, als Konservierungsmittel nur Phenoxyethanol und als Stabilisatoren nur Methylpropandiol und Caprylyl Glycol.

Erfindungsgemäß vorteilhaft ist ebenfalls, wenn zusätzlich ein oder mehrere Feuchthaltemittel in die erfindungsgemäße Zubereitung eingearbeitet werden. Feuchthaltemittel können der Haut Feuchtigkeit verleihen und/oder dazu beitragen, einen Feuchtigkeitsverlust der Haut zu verhindern. Bei den Feuchthaltemitteln handelt es sich um hygroskopische Substanzen, die Wasser binden können. Dieses Wasserbindevermögen beruht auf hydrophilen Gruppen, wie Hydroxylgruppen, aber auch Amingruppen und Carboxylgruppen. Feuchthaltemittel sind beispielsweise Glycerin, Biosaccaride Gum-1, Pyrrolidoncarbonsäure, Harnstoff, Sorbitol, Xylitol, Aloe Vera Gel und andere mehr. Erfindungsgemäß bevorzugt ist der Einsatz von Glycerin.

Die unter Stabilisatoren genannten Verbindungen Propylenglycol, Ethylhexylglycerin, 1,2-Hexanediol, Methylpropanediol, Butylene Glycol, Caprylyl Glycol, Glyceryl Caprylate und Pentylene Glycol haben neben ihrer stabilisierenden Wirkung auch eine feuchthaltende Wirkung.

Wenn in dem erfindungsgemäßen Reinigungsgel zusätzlich ein oder mehrere Feuchthaltemittel enthalten sind, so liegt/en das oder die Feuchthaltemittel mit einem Gesamtgehalt von 0,01 bis 30 Gew.-%, bevorzugt 5 bis 20 Gew.-%, bezogen auf das Gesamtgewicht der Zubereitung, vor.

Die erfindungsgemäße Zubereitung kann kosmetische Hilfsstoffe enthalten, wie sie üblicherweise in solchen Zubereitungen verwendet werden, z. B. Komplexbildner, Parfüme, Substanzen zum Steigern des Schäumens, Füllstoffe, die das Hautgefühl verbessern, Fette, Öle, Wachse oder andere übliche Bestandteile einer kosmetischen oder dermatologischen Formulierung, wie beispielsweise Schaumstabilisatoren, Elektrolyte und andere mehr.

Die erfindungsgemäße Zubereitung kann nach jeder in der kosmetischen Industrie üblichen und für diese Zubereitungsform geeigneten Methode hergestellt werden.

Das erfindungsgemäße Reinigungsgel ist transparent. Die Transparenz wurde mithilfe einer Transmissionsmessung bestimmt. Die Messung wurde mit einem handelsüblichen UV-Vis-Spektrometer bei 420nm durchgeführt; die Messung erfolgte in 1cm-Einmalküvetten gegen Aqua dest. als Referenz. Eine Zubereitung ist im Sinne der Erfindung transparent, wenn die Transmissionswerte mindestens 80 % in Bezug auf den Referenzwert erreichen.

Um eine problemlose Entnahme aus einer Tube zu gewährleisten, weist das erfindungsgemäße Reinigungsgel vorteilhaft eine Schubspannung an der Fließgrenze von mindestens 10 Pa und maximal 50 Pa auf.

Als Fließgrenze im Sinne der vorliegenden Schrift wird die kritische Schubspannung der Fließkurve angesehen.

Gemessen wird die Fließkurve auf einem schubspannungsgesteuerten Rheometer Typ SR-2000 der Firma Rheometric Scientific bei 25°C ± 1°C mit 25 mm Platte/Platte Geometrie bei einem Spalt von 1 mm, wobei strukturschonend befüllt wird. Es wird eine geeignete konstante Schubspannungszeitrampe von 40 Pa/min vorgegeben und vor dem Test eine entsprechende Strukturerholungszeit eingehalten und die kritische Schubspannung im Maximum der Fließkurve angegeben.

Das Produkt wird vorsichtig mit einem Löffelspatel entnommen und in das Messgerät eingebracht. Dabei sollte das Produkt nicht geschert werden, um eine Zerstörung oder Beeinflussung der Strukturen zu vermeiden.

Das erfindungsgemäße Reinigungsgel ist milde zur Haut. Um dies zu belegen, wurde ein RBC-Assay durchgeführt.

Der Standard-RBC-Assay (10 Minuten Inkubation) dient zur Abschätzung von in vivo Augenschleimhautreizpotentialen von Tensiden und tensidhaltigen Produkten.

### 1. Hämolvse:

Ein definiertes Aliquot isolierter Kalbserythrozyten wird mit einer Reihe steigender Konzentrationen des zu untersuchenden WAS-Prüfmusters (Stammlösung mit Formulierungen 1:100 w/v bzw. für Rohstoffe 0.1 % Aktivgehalt in PBS) für 10 Minuten unter Schütteln bei Raumtemperatur (RT) inkubiert. Nach Zentrifugation werden die gewonnenen Überstände photometrisch auf ihren Gehalt an freigesetztem Hämoglobin (HbO2) bei 530 nm analysiert. Daraus wird der relative Hämolysegrad berechnet und die Konzentrations-Response-Kurve mit dem H50-Wert [µl/ml] als Kenngröße bestimmt. Dieser gibt die Konzentration des Prüfmusters an, bei der 50% des Hämoglobins freigesetzt werden.

### 2. HbO₂-Denaturierung:

Ein definiertes Aliquot isolierter Kalbserythrozyten wird mit einer fixen Konzentration des Prüfmusters (1% w/v bzw. 0.1% Aktivgehalt) für 10 Minuten unter Schütteln bei RT inkubiert und dann zentrifugiert. Die Änderung der spektralen Absorption bei 575 nm und 540 nm wird im Vergleich zum nativen HbO2 gemessen. Aus dem Verhältnis der Absorptionswerte zueinander wird der Denaturierungsindex DI [%] berechnet. Als 100%-Standard dient Na-Laurylsulfat (0.1% Aktivgehalt).

### 3. L/D-Quotient:

Der Quotient stellt das Verhältnis der Kenngrößen von Hämolyse (H50) und Denaturierung (DI) dar und wird zur Charakterisierung und Klassifizierung der untersuchten Prüfmuster verwendet. Zur weitergehenden Information wird auf das Dokument Pape, W.J.W. et al., Molecular Toxicology, 1,525-536, 1987, verwiesen.

Die getesteten Reinigungsgele wiesen in diesem empfindlichen Test, der auf eine Bestimmung des Augenschleimhautreizpotentials ausgerichtet ist, Werte von 3,06 und 5,43 (L/D-

Quotient) auf, also "moderately irritant". Dies sind für Reinigungszubereitungen sehr gute Werte.

Die im genannten Test ermittelten Werte können folgendermaßen eingeordnet werden:

| L/D-Quotient | Bewertung |
|---|---|
| > 100 | Non-irritant |
| > 10 | Slightly irritant |
| > 1 | Moderately irritant |
| > 0,1 | irritant |
| < 0,1 | Strongly irritant/corrosive |

Aus den vorstehenden Messungen und Ergebnissen wird deutlich, dass sehr transparente Reinigungsgele zur Verfügung gestellt werden, die aufgrund der günstigen rheologischen Eigenschaften problemlos aus einer Tube entnommen werden können und zudem für Reinigungszubereitungen sehr milde sind. Diese Reinigungsgele sind aufgrund dieser Eigenschaften auch gut geeignet, der Rasurvorbereitung zu dienen, also als Rasiergele.

### Beispiele:

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen. Die Angaben beziehen sich auf die jeweiligen Aktivgehalte.

| **INCI-Bezeichnung** | **1** | **2** | **3** | **4** | **5** |
|---|---|---|---|---|---|
| Sodium Cocoyl Glutamate | 3 | 3 | 0 | 0 | 3 |
| Parfüm | 0 | 0 | 0,35 | 0 | 0,35 |
| Caprylyl Glycol | 0,25 | 0,25 | 0,25 | 0,25 | 0,25 |
| Glycerin | 5 | 5 | 5 | 5 | 5 |
| Methylpropanediol | 0 | 0 | 3 | 3 | 0 |
| Citronensäure | 0 | 0 | 0,2 | 0,2 | 0 |
| Phenoxyethanol | 0 | 0,8 | 0,8 | 0,8 | 0 |
| Alcohol Denat | 10,0 | 10 | 0 | 0 | 10 |
| Hydroxypropyl Guar | 0,465 | 0,465 | 0,465 | 0,465 | 0,465 |
| Xanthan Gum | 1,1 | 1,1 | 1,1 | 1,1 | 1,1 |
| Wasser | Ad 100 | Ad 100 | Ad 100 | Ad 100 | Ad 100 |
| Cocamidopropylbetain | 1,68 | 1,68 | 1,6 | 1,6 | 1,68 |
| Sodium Lauroyl Sarcosinate | 1,5 | 1,5 | 4,5 | 4,5 | 1,5 |
| Coco-Glucoside | 1,54 | 1,54 | 1,43 | 1,43 | 1,54 |
| | | | | | |
| Transmission bei 420 nm | 84,0 | | 83,1 | 85,3 | 83,2 |
| | | | | | |
| RBC-Test: L/D-Quotient | | 5,43 | 3,06 | | |
| | | | | | |
| Schubspannung [Pa] | 20,1 | | 13,2 | 16,0 | 20,8 |

## Patentansprüche

1. Kosmetisches Reinigungsgel, enthaltend
• wenigstens ein Aminosäure-Tensid, bevorzugt ausgewählt aus wenigstens einem Natrium Acyl-Sarkosinat und/oder wenigstens einem Natrium/Dinatrium Acyl Glutamat,
• wenigstens ein weiteres Tensid, das kein Aminosäure-Tensid ist, bevorzugt ausgewählt aus wenigstens einem Alkylpropylbetain und/oder wenigstens einem Alkylpolyglucosid,
• eine Kombination der Polymere Xanthan Gum und Hydroxypropyl Guar.

2. Reinigungsgel nach Anspruch 1, **dadurch gekennzeichnet, dass** der Wassergehalt von 50 bis 95 Gew.-%, bevorzugt von 65 bis 90 Gew.-% beträgt, bezogen auf das Gesamtgewicht des Reinigungsgels.

3. Reinigungsgel nach Anspruch 1und/oder 2, **dadurch gekennzeichnet, dass** das Reinigungsgel keine synthetischen Polymere, keine Parabene und/oder keine Sulfat-haltigen Verbindungen enthält.

4. Reinigungsgel nach wenigstens einem der vorstehenden Ansprüche, **dadurch gekennzeichnet, dass** das wenigstens eine Natrium Acylsarkosinat mit einem Gehalt von 0,5 bis 10,0 Gew.-%, bevorzugt 2,0 bis 6,0 Gew.-% vorliegt, bezogen auf das Gesamtgewicht des Gels

5. Reinigungsgel nach wenigstens einem der vorstehenden Ansprüche, **dadurch gekennzeichnet, dass** das Natrium Acyl-Sarkosinate Natrium Lauroylsarkosinat ist.

6. Reinigungsgel nach wenigstens einem der vorstehenden Ansprüche, **dadurch gekennzeichnet, dass** das wenigstens ein Natrium/Dinatrium Acylglutamat mit einem Gesamtgehalt von 0,5 bis 10,0 Gew.-%, bevorzugt 0,5 bis 5,0 Gew.-% vorliegt, bezogen auf das Gesamtgewicht des Gels.

7. Reinigungsgel nach wenigstens einem der vorstehenden Ansprüche, **dadurch gekennzeichnet, dass** das Natrium/Dinatrium Acylglutamat Dinatrium Cocoyl Glutamat ist.

8. Reinigungsgel nach wenigstens einem der vorstehenden Ansprüche, **dadurch gekennzeichnet, dass** wenigstens ein Natrium Acylsarkosinat und wenigstens ein Natrium/Dinatrium Acylglutamat vorliegen, bevorzugt mit einem Gesamtgewicht von 1,0 bis 12,0 Gew.-%, bevorzugt 2,5 bis 11,0 Gew.-%, bezogen auf das Gesamtgewicht des Gels.

9. Reinigungsgel nach wenigstens einem der vorstehenden Ansprüche, **dadurch gekennzeichnet, dass** das wenigstens eine Alkylamidopropylbetain mit einem Gesamtgewicht von 0,5 bis 15 Gew.-%, bevorzugt 2,5 bis 12 Gew.-% vorliegt, bezogen auf das Gesamtgewicht des Gels

10. Reinigungsgel nach wenigstens einem der vorstehenden Ansprüche, **dadurch gekennzeichnet, dass** Alkylamidopropylbetaine Cocamidopropylbetaine ist.

11. Reinigungsgel nach wenigstens einem der vorstehenden Ansprüche, **dadurch gekennzeichnet, dass** das wenigstens eine Alkylpolyglucosid mit einem Gesamtgewicht von 0,1 bis 10,0 Gew.-%, bevorzugt 1,0 bis 2,5 Gew.-% vorliegt, bezogen auf das Gesamtgewicht des Gels.

12. Reinigungsgel nach wenigstens einem der vorstehenden Ansprüche, **dadurch gekennzeichnet, dass** das wenigstens eine Alkylpolyglucosid aus der Gruppe Coco-Glucosid, Decylglucosid, Laurylglucosid und/oder Caprylyl/Capryl Glucosid ausgewählt wird, bevorzugt aus der Gruppe Cocoglucosid und/oder Caprylyl/Capryl Glucosid.

13. Reinigungsgel nach wenigstens einem der vorstehenden Ansprüche, **dadurch gekennzeichnet, dass** wenigstens ein Alkylamidopropylbetain und wenigstens ein Alkylpolyglucosid enthalten sind, bevorzugt mit einem Gesamtgewicht im Bereich von 0,6 bis 15,0 Gew.-%, bevorzugt 2,6 bis 12,0 Gew.-%, bezogen auf das Gesamtgewicht des Gels.

14. Reinigungsgel nach wenigstens einem der vorstehenden Ansprüche, **dadurch gekennzeichnet, dass** die Kombination der Polymere Xanthan Gum und Hydroxypropyl Guar mit einem Gesamtgewicht von 0,9 bis 3,0 Gew.-%, bevorzugt 1,0 bis 2,5 Gew.-% enthalten ist, bezogen auf das Gesamtgewicht des Gels.

15. Reinigungsgel nach wenigstens einem der vorstehenden Ansprüche, **dadurch gekennzeichnet, dass** Phenoxyethanol enthalten ist, bevorzugt mit einem Gehalt von 0,4 bis 0,95 Gew.-%, bevorzugt 0,6 bis 0,9 Gew.-% vor, bezogen auf das Gesamtgewicht des Gels.

16. Reinigungsgel nach wenigstens einem der vorstehenden Ansprüche, **dadurch gekennzeichnet, dass** wenigstens ein Stabilisator ausgewählt aus der Gruppe Propylenglycol, Ethylhexylglycerin, 1,2-Hexanediol, Methylpropandiol, Butylene Glycol, Caprylyl Glycol, Glyceryl Caprylate, Hydroxyacetophenone, Pentylene Glycol, Ethanol und/oder Alkohol denat., bevorzugt aus der Gruppe Methylpropandiol, Caprylyl Glycol und/oder Alkohol denat. enthalten ist

17. Reinigungsgel nach wenigstens einem der vorstehenden Ansprüche, **dadurch gekennzeichnet, dass** der wenigstens eine Stabilisator mit einem Gesamtgewicht von 0,2 bis 10,0 Gew.-% vorliegt, bezogen auf das Gesamtgewicht des Gels.

18. Reinigungsgel nach wenigstens einem der vorstehenden Ansprüche, **dadurch gekennzeichnet, dass** der pH-Wert des erfindungsgemäßen Reinigungsgels im neutralen oder Haut-neutralen Bereich liegt.

19. Reinigungsgel nach Anspruch 18, **dadurch gekennzeichnet, dass** der pH-Wert 7 beträgt und als Stabilisatoren nur Alkohol denat. und Caprylyl Glycol und keine weiteren Konservierungsmittel enthalten sind.

20. Reinigungsgel nach Anspruch 18, **dadurch gekennzeichnet, dass** der pH-Wert 5,5, beträgt und als Aminosäuretensid nur Natrium Lauroyl Sarcosinate, als weitere Tenside nur Cocamidopropylbetaine und Cocoglucosid, als Konservierungsmittel nur Phenoxyethanol und als Stabilisatoren nur Methylpropandiol und Caprylyl Glycol enthalten sind.

21. Reinigungsgel nach wenigstens einem der vorstehenden Ansprüche, **dadurch gekennzeichnet, dass** der Transmissionswert mindestens 80 % des Referenzwertes beträgt, wobei die Messung bei 420 nm durchgeführt wird, der Referenzwert mit Aqua dest. ermittelt wird und die Messküvette eine 1cm-Einmalküvette ist.

22. Reinigungsgel nach wenigstens einem der vorstehenden Ansprüche, **dadurch gekennzeichnet, dass** das Reinigungsgel eine Schubspannung an der Fließgrenze von mindestens 10 Pa und maximal 50 Pa aufweist, wobei eine Fließkurve auf einem schubspannungsgesteuerten Rheometer Typ SR-2000 der Firma Rheometric Scientific bei 25°C ± 1°C mit 25 mm Platte/Platte Geometrie bei einem Spalt von 1 mm gemessen wird, wobei eine geeignete konstante Schubspannungszeitrampe von 40 Pa/min vorgegeben und die Schubspannung im Maximum der Fließkurve bestimmt wird.
